# EUROPEAN PATENT APPLICATION

(11) **EP 4 306 523 A2**
(43) Date of publication of application: **17.01.2024**
(21) Application number: 23188546.8
(22) Date of filing: 05.09.2019
(51) Int. Cl.: C07D 471/04

(54) **IMIDAZOQUINOLINE COMPOUNDS AND USES THEREOF**

(30) Priority: 07.09.2018 US 201862728556 P
(62) Divisional of application: 19858183.7
(71) Applicant: Birdie Biopharmaceuticals, Inc., George Town, Grand Cayman KY1-1002 (KY)
(72) Inventor: YANG, Lihu, New Jersey, 08837 (US)
(74) Representative: Snaith, James Michael

(57) **Abstract**

Provided herein are imidazoquinoline derivatives, or pharmaceutically acceptable salts thereof, that are agonists of toll-like receptors 7 and 8 (TLR7/8). Also provided herein are compositions and pharmaceutical compositions comprising the imidazoquinoline derivatives, or pharmaceutically acceptable salts thereof, provided herein. Also provided herein are methods of use of the imidazoquinoline derivatives, or pharmaceutically acceptable salts thereof, provided herein to treat various diseases, such as viral, cancer, and allergic diseases, in a subject in need thereof by administering a therapeutically effective amount of the imidazoquinoline derivative, or pharmaceutically acceptable salt thereof, to the subject.

## Description

### RELATED APPLICATIONS

This application claims the benefit of U.S. Provisional Patent Application No. 62/728,556, filed September 7, 2018, the entire content of which is incorporated herein by reference.

### BACKGROUND

Toll-like receptors (TLRs) play a fundamental role in pathogen recognition and activation of innate immunity. TLR7 and TLR8 are toll-like receptors 7 and 8 respectively, and they lie in close proximity to each other on the human X chromosome. Both TLR7 and TLR8 recognize single-stranded RNA of viruses such as HIV and HCV. TLR7 has been shown to play a significant role in the pathogenesis of autoimmune disorders such as Systemic Lupus Erythematosus (SLE) as well as in antiviral immunity regulation. Genetic variants in TLR8 have recently been linked to susceptibility to pulmonary tuberculosis. TLR7 is functional both in human and mouse, while TLR8 is only functional in human, but it seems to counteract TLR7 activity. One benefit of TLR7/8 agonists as immune response enhancers is their simultaneous stimulation of several cell types. TLR7 and TLR8 are expressed mostly on immune cells such as antigen presenting cells, including plasmacytoid dendritic cells (pDC) and myeloid dendritic cells (mDC), as well as natural killer cells, and macrophages. TLR7/8 activation on pDCs and mDCs results in induction and release of type I interferons (IFN), tumor necrosis factor alpha (TNFα), and interleukin 12 (IL-12), which is an important step for the initiation of innate and adaptive immunities to kill cancer cells. Accordingly, there is a need to develop small molecule agonists of TLR7 and TLR8 as both antiviral and antitumor compounds.

### SUMMARY

Provided herein are compounds that are imidazoquinoline derivatives, or pharmaceutically acceptable salts thereof. The compounds are agonists of toll-like receptors 7 and 8 (TLR7/8). The compounds may be used to treat viral infection (e.g., hepatitis C (HCV)), cancer (e.g., a HER2 positive cancer), allergic disease, or a combination thereof, in a subject need thereof by administering a therapeutically effective amount of an imidazoquinoline derivative provided herein to the subject.

In one aspect, provided herein are compounds of Formula (I): 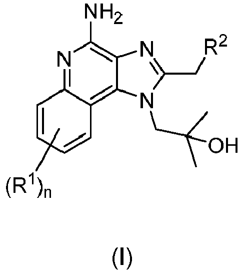 or a pharmaceutically acceptable salt thereof,
wherein
each occurrence of R¹ is, independently, F, Cl, Br, or I;
R² is -O-(C₃₋₅ cycloalkyl), -O-(C₁₋₃ alkyl), -O-(C₁₋₃ haloalkyl), -S-(C₁₋₃ alkyl),-S(O)(C₁₋₃ alkyl), or -S(O₂)(C₁₋₃ alkyl); and
n is 0, 1, 2, 3, or 4.

Some embodiments include a pharmaceutical dosage form comprising a compound described herein. Compounds or compositions described herein may be used for activating TLR7/8. In addition to use as a stand-alone immunotherapeutic agent, such as an antiviral and anticancer agent, compounds or compositions described herein may be used as adjuvants in cancer vaccine or adoptive T cell transfer protocols.

Some embodiments include a method of treating a disease or disorder associated with a TLR7/8 agonist comprising administering an effective amount of a compound described herein to a mammal in need thereof.

Some embodiments include use of a compound described herein in the manufacture of a medicament for the treatment of a disease or disorder associated with a TLR7/8 agonist.

### BRIEF DESCRIPTION OF THE DRAWINGS

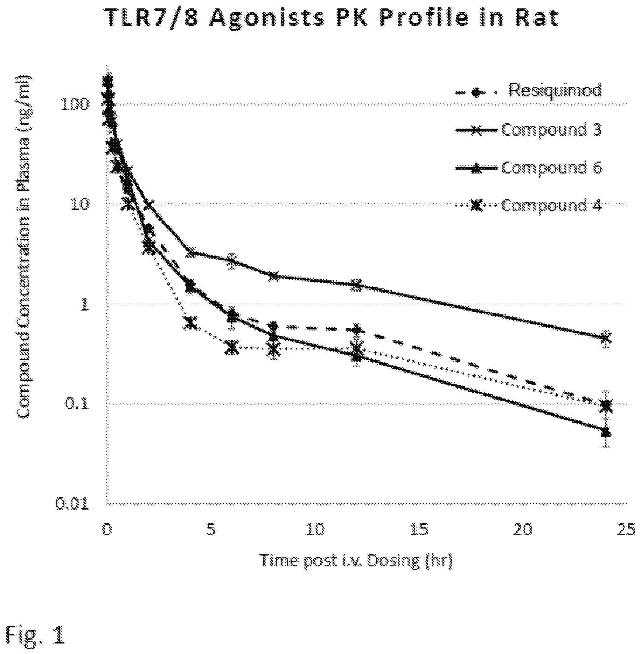

Fig. 1 shows PK profiles in rats as mean concentration versus time curves for TLR7/8 agonists as described in Example 15.

### DETAILED DESCRIPTION

Listed below are definitions of various terms used in the present disclosure. These definitions apply to the terms as they are used throughout this specification and claims, unless otherwise limited in specific instances, either individually or as part of a larger group.

As used herein, the article "a" or "an" refers to one or to more than one (i.e. to at least one) of the grammatical object of the article. By way of example, "an element" means one element or more than one element. Furthermore, use of the term "including" as well as other forms, such as "include", "includes," and "included," is not limiting.

As used herein, the term "administering" refers to administration of the compounds provided herein to a cell or a subject as needed to achieve the desired effect.

As used herein, the term "alkyl," by itself or as part of another substituent means, unless otherwise stated, a straight or branched chain hydrocarbon having the number of carbon atoms designated (i.e., C₁₋₃ means one to three carbon atoms) and includes straight or branched chain substituent groups.

As used herein, the term "composition" or "pharmaceutical composition" refers to a mixture of at least one compound-useful as described herein-with a pharmaceutically acceptable carrier. The pharmaceutical composition facilitates administration of the compound to a patient or subject. Multiple techniques of administering a compound exist in the art including, but not limited to, intravenous, oral, aerosol, parenteral, ophthalmic, pulmonary, rectal, subcutaneous, and topical administration.

As used herein, the term "controlling the disease or disorder" is used to mean changing the activity of one or more kinases to affect the disease or disorder.

As used herein, the term "cycloalkyl" refers to a mono cyclic non-aromatic radical, wherein each of the atoms forming the ring (i.e., skeletal atoms) is a carbon atom. In one embodiment, the cycloalkyl group is saturated or partially unsaturated. Cycloalkyl groups include groups having 3 to 5 ring atoms (C₃₋₅ cycloalkyl).

As used herein, the term "disease or disorder associated with kinase activity" refers to a disease, condition or disorder treatable, in whole or in part, by inhibition of one or more kinases.

As used herein, the term "effective amount," "pharmaceutically effective amount" or "therapeutically effective amount" refers to a nontoxic but sufficient dosage amount of an agent (e.g., the compounds or compositions provided herein) to provide the desired biological result, which result may be reduction or alleviation, or both, of the signs, symptoms, or causes of a disease, or any other desired alteration of a biological system including influencing, reducing or inhibiting the activity of or preventing activation of a kinase (e.g., modulating kinase activity). An appropriate therapeutic amount in any individual case may be determined by one of ordinary skill in the art using routine experimentation. These terms as used herein may also refer to an amount effective at bringing about a desired *in vivo* effect in an animal-where in some embodiments, the animal is a human-including, but not limited to, uveitis, reduction in intraocular pressure, or dry eye.

As used herein, the term "excipient" refers to physiologically compatible additives useful in preparation of a pharmaceutical composition. Examples of pharmaceutically acceptable carriers and excipients can, for example, be found in Remington Pharmaceutical Science, 16^{th} Ed.

As used herein, the term "haloalkyl" refers to an alkyl group independently substituted with one or more fluorine, chlorine, bromine, or iodine atoms. In some embodiments, the alkyl group is independently substituted with one or more fluorine, chlorine, or bromine atoms. In some embodiments, the alkyl group is independently substituted with one or more fluorine or chlorine atoms.

As used herein, the term "subject," "patient" or "individual" refers to a human or a non-human mammal. Non-human mammals include, for example, livestock and pets, such as ovine, bovine, porcine, canine, feline, and murine mammals. In some embodiments, the patient, subject, or individual is human.

As used herein, the term "pharmaceutically acceptable" refers to a material that does not abrogate the biological activity or properties of the compound, and is relatively non-toxic, i.e. the material may be administered to an individual without causing undesirable biological effects or interacting in a deleterious manner with any of the components of the composition in which it is contained.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the invention within or to the patient such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the invention, and not injurious to the patient. As used herein, "pharmaceutically acceptable carrier" also includes any and all coatings, antibacterial and antifungal agents, and absorption delaying agents, and the like that are compatible with the activity of the compound useful within the invention, and are physiologically acceptable to the patient. The term "pharmaceutically acceptable carrier" may further include a pharmaceutically acceptable salt of the compound useful within the invention. Other additional ingredients that may be included in the pharmaceutical compositions used in the practice of the invention are described, for example, in Remington's Pharmaceutical Sciences (Genaro, Ed., Mack Publishing Co., 1985, Easton, Pa.), which is incorporated herein by reference. The "pharmaceutically acceptable carrier" is useful for the preparation of a pharmaceutical composition that is: generally compatible with the other ingredients of the composition, not deleterious to the recipient, and neither biologically nor otherwise undesirable. "A pharmaceutically acceptable carrier" includes one or more than one carrier. Embodiments include carriers for topical, ocular, parenteral, intravenous, intraperitoneal intramuscular, sublingual, nasal or oral administration. "Pharmaceutically acceptable carrier" also includes agents for preparation of aqueous dispersions and sterile powders for injection or dispersions.

As used herein, the term "pharmaceutically acceptable salt" refers to derivatives of the compounds provided herein wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the compounds provided herein include the conventional non-toxic salts of the parent compound formed, for example, from non-toxic inorganic or organic acids. The pharmaceutically acceptable salts of the compounds provided herein can be synthesized from the parent compound which contains a basic or acidic moiety by conventional chemical methods. Generally, such salts can be prepared by combining the free acid or base forms of these compounds with a stoichiometric amount of the appropriate base or acid in water or in an organic solvent, or in a mixture of the two; generally, nonaqueous media such as ether, ethyl acetate, ethanol, isopropanol, or acetonitrile may be used. Lists of suitable salts are found in Remington's Pharmaceutical Sciences, 17th ed., Mack Publishing Company, Easton, Pa., 1985, p. 1418 and Journal of Pharmaceutical Science, 66, 2 (1977), each of which is incorporated herein by reference in its entirety.

As used herein, the term "prevent" or "prevention" refers to no disorder or disease development if none had occurred, or no further disorder or disease development if there had already been development of the disorder or disease. Also considered is the ability of one to prevent some or all of the symptoms associated with the disorder or disease.

As used herein, the term "treatment" or "treating" refers to the application or administration of a therapeutic agent, i.e. a compound provided herein, to a patient, or application or administration of a therapeutic agent to an isolated tissue or cell line from a patient (e.g., for diagnosis or ex vivo applications), who has a disease, a symptom of the disease or the potential to develop the disease, with the purpose to heal, alleviate, relieve, alter, remedy, ameliorate, improve or affect the disease, the symptoms of the disease, or the potential to develop the disease. Such treatments may be specifically tailored or modified, based on knowledge obtained from the field of pharmacogenomics.

### COMPOUNDS

In one aspect, provided herein are compounds that are imidazoquinoline derivatives. In one embodiment, the compound is a compound of Table 1, or a pharmaceutically acceptable salt thereof.

**Table 1.**

| **Compound #** | **Structure** |
|---|---|
| 1 | 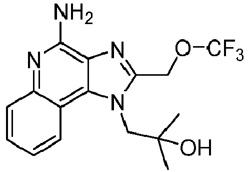 |
| 2 | 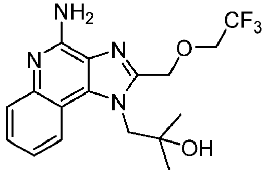 |
| 3 | 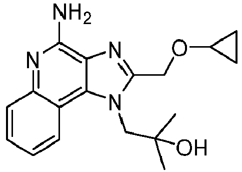 |
| 4 | 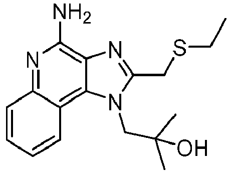 |
| 5 | 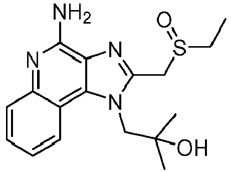 |
| 6 | 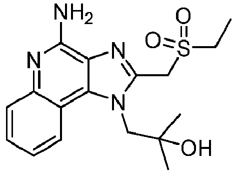 |
| 7 | 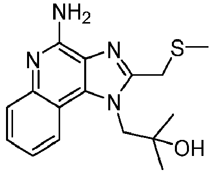 |
| 8 | 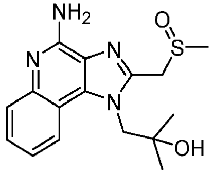 |
| 9 | 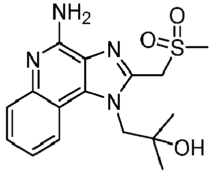 |
| 10 | 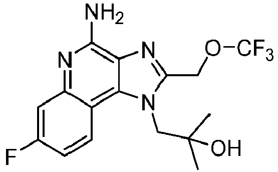 |
| 11 | 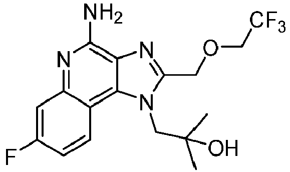 |
| 12 | 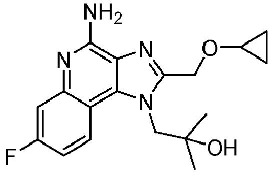 |
| 13 | 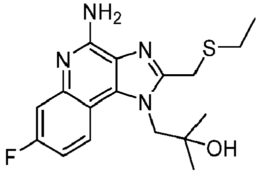 |
| 14 | 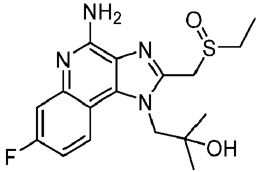 |
| 15 | 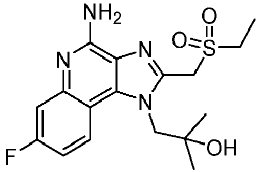 |
| 16 | 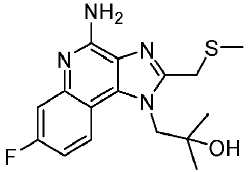 |
| 17 | 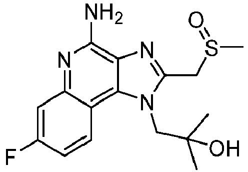 |
| 18 | 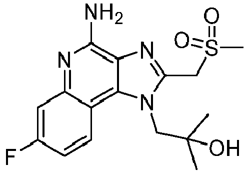 |

In another aspect, provided herein are compounds of Formula (I): 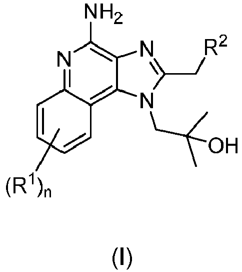 or a pharmaceutically acceptable salt thereof,
wherein
each occurrence of R¹ is, independently, F, Cl, Br, or I;
R² is -O-(C₃₋₅ cycloalkyl), -O-(C₁₋₃ alkyl), -O-(C₁₋₃ haloalkyl), -S-(C₁₋₃ alkyl),-S(O)(C₁₋₃ alkyl), or -S(O₂)(C₁₋₃ alkyl); and
n is 0, 1, 2, 3, or 4.

In some embodiments, the compound of Formula (I) is a compound of Formula (II): 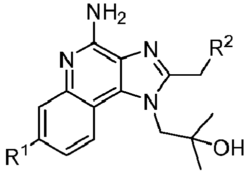 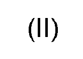 or a pharmaceutically acceptable salt thereof.

In some embodiments, the compound of Formula (I) is a compound of Formula (III): 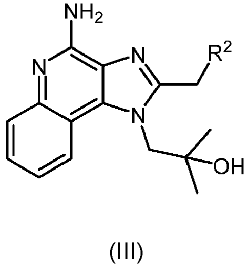 or a pharmaceutically acceptable salt thereof.

In some embodiments, R¹ is F, Cl, Br, or I. In some embodiments, R¹ is F. In some embodiments, R¹ is H or F. In some embodiments, R¹ is H (i.e. n is 0). In some embodiments, R¹ is H, F, Cl, Br, or I.

In some embodiments, R² is -O-(C₃₋₅ cycloalkyl), -O-(C₁₋₃ alkyl), -O-(C₁₋₃ monohaloalkyl), -O-(C₁₋₃ dihaloalkyl), -O-(C₁₋₃ trihaloalkyl), -S-(C₁₋₃ alkyl), -S(O)(C₁₋₃ alkyl), or -S(O₂)(C₁₋₃ alkyl). In some embodiments, R² is -O-CF₃, -O-CH₂-CF₃, -O-cyclopropyl, —S-methyl, —S-ethyl, —S(O)-methyl, —S(O)-ethyl, -S(O₂)-methyl, or-S(Oz)-ethyl. In some embodiments the haloalkyl halogen is independently F, Cl, Br, or I. In some embodiments the haloalkyl halogen is F.

In some embodiments, R² is -O-(C₃₋₅ cycloalkyl), -O-(C₁₋₃ alkyl), -S-(C₁₋₃ alkyl), -S(O)(C₁₋₃ alkyl), or -S(O₂)(C₁₋₃ alkyl). In some embodiments, R² is -O-(C₃₋₅ cycloalkyl), -S-(C₁₋₃ alkyl), -S(O)(C₁₋₃ alkyl), or -S(O₂)(C₁₋₃ alkyl). In some embodiments, R² is -O-cyclopropyl, —S-methyl, —S-ethyl, —S(O)-methyl, —S(O)-ethyl, -S(O₂)-methyl, or-S(O₂)-ethyl.

In some embodiments, R² is -O-(C₃₋₅ cycloalkyl). In some embodiments, R² is -O-(C₃₋₅ cycloalkyl), and R¹ is F, Cl, Br, or I. In some embodiments, R² is -O-(C₃₋₅ cycloalkyl), and R¹ is H or F. In some embodiments, R² is -O-(C₃₋₅ cycloalkyl), and R¹ is H. In some embodiments, R² is -O-(C₃₋₅ cycloalkyl), and R¹ is F.

In some embodiments, R² is -O-(C₃₋₅ cycloalkyl), -S-(C₁₋₃ alkyl), -S(O)(C₁₋₃ alkyl), or -S(O₂)(C₁₋₃ alkyl), and R¹ is F, Cl, Br, or I. In some embodiments, R² is -O-(C₃₋₅ cycloalkyl), -S-(C₁₋₃ alkyl), -S(O)(C₁₋₃ alkyl), or -S(O₂)(C₁₋₃ alkyl), and R¹ is H or F. In some embodiments, R² is -O-(C₃₋₅ cycloalkyl), -S-(C₁₋₃ alkyl), -S(O)(C₁₋₃ alkyl), or-S(O₂)(C₁₋₃ alkyl), and R¹ is F. In some embodiments, R² is -O-(C₃₋₅ cycloalkyl), -S-(C₁₋₃ alkyl), -S(O)(C₁₋₃ alkyl), or -S(O₂)(C₁₋₃ alkyl), and R¹ is H.

In some embodiments, R² is -S-(C₁₋₃ alkyl), -S(O)(C₁₋₃ alkyl), or -S(O₂)(C₁₋₃ alkyl), and R¹ is F, Cl, Br, or I. In some embodiments, R² is -S-(C₁₋₃ alkyl), -S(O)(C₁₋₃ alkyl), or -S(O₂)(C₁₋₃ alkyl), and R¹ is H or F. In some embodiments, R² is -S-(C₁₋₃ alkyl), -S(O)(C₁₋₃ alkyl), or -S(O₂)(C₁₋₃ alkyl), and R¹ is F. In some embodiments, R² is -S-(C₁₋₃ alkyl), -S(O)(C₁₋₃ alkyl), or -S(O₂)(C₁₋₃ alkyl), and R¹ is H.

In some embodiments, R² is —O-cyclopropyl, —S-methyl, —S-ethyl, —S(O)-methyl, —S(O)-ethyl, -S(O₂)-methyl, or -S(O₂)-ethyl, and R¹ is F, Cl, Br, or I. In some embodiments, R² is —O-cyclopropyl, —S-methyl, —S-ethyl, —S(O)-methyl, —S(O)-ethyl,-S(O₂)-methyl, or -S(O₂)-ethyl, and R¹ is H or F. In some embodiments, R² is -O-cyclopropyl, —S-methyl, —S-ethyl, —S(O)-methyl, —S(O)-ethyl, -S(O₂)-methyl, or-S(O₂)-ethyl, and R¹ is H. In some embodiments, R² is —O-cyclopropyl, —S-methyl, -S-ethyl, —S(O)-methyl, —S(O)-ethyl, -S(O₂)-methyl, or -S(O₂)-ethyl, and R¹ is F.

In some embodiments, n is 0. In some embodiments, n is 1. In some embodiments, n is 2. In some embodiments, n is 1, 2, 3, or 4. In some embodiments, n is 0 or 1.

In some embodiments, the compound is: 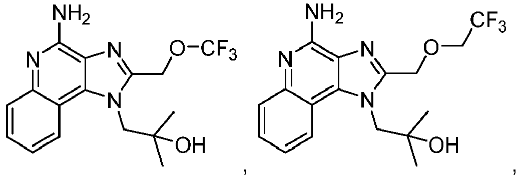 or a pharmaceutically acceptable salt thereof.

In some embodiments, the compound is: 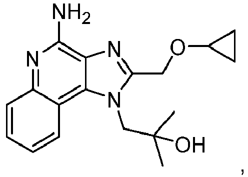 or a pharmaceutically acceptable salt thereof.

In some embodiments, the compound is: 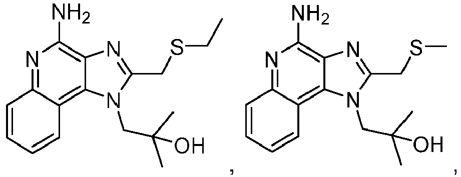 or a pharmaceutically acceptable salt thereof.

In some embodiments, the compound is: 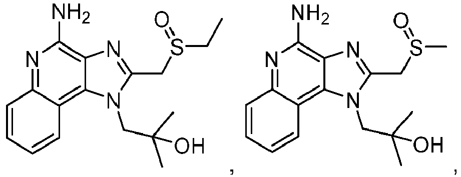 or a pharmaceutically acceptable salt thereof.

In some embodiments, the compound is: 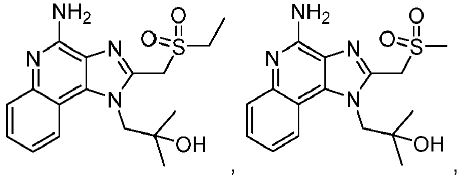 or a pharmaceutically acceptable salt thereof.

In some embodiments, the compound is: 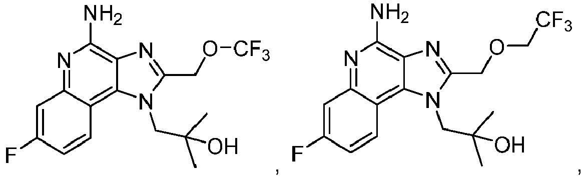 or a pharmaceutically acceptable salt thereof.

In some embodiments, the compound is: 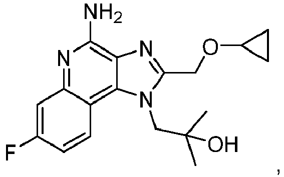 or a pharmaceutically acceptable salt thereof.

In some embodiments, the compound is: 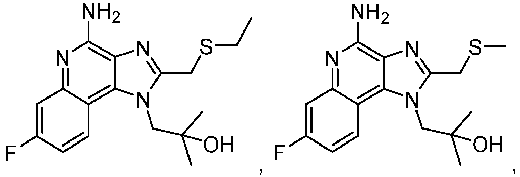 or a pharmaceutically acceptable salt thereof.

In some embodiments, the compound is: 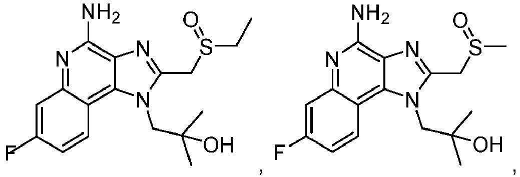 or a pharmaceutically acceptable salt thereof.

In some embodiments, the compound is: 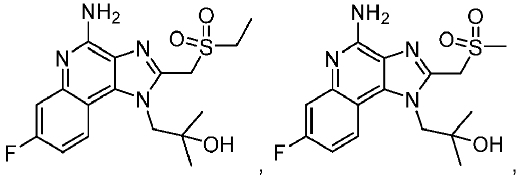 or a pharmaceutically acceptable salt thereof.

Compounds described herein also include isotopically-labeled compounds wherein one or more atoms is replaced by an atom having the same atomic number, but an atomic mass or mass number different from the atomic mass or mass number usually found in nature. Examples of isotopes suitable for inclusion in the compounds described herein include and are not limited to ²H, ³H, ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, ³²P, and ³⁵S. In one embodiment, isotopically-labeled compounds are useful in drug and/or substrate tissue distribution studies. In another embodiment, substitution with heavier isotopes such as deuterium affords greater metabolic stability (for example, increased *in vivo* half-life or reduced dosage requirements). In yet another embodiment, substitution with positron emitting isotopes, such as ¹¹C, ¹⁸F, ¹⁵O and ¹³N, is useful in Positron Emission Topography (PET) studies for examining substrate receptor occupancy. Isotopically-labeled compounds are prepared by any suitable method or by processes using an appropriate isotopically-labeled reagent in place of the non-labeled reagent otherwise employed.

In one embodiment, compounds described herein are labeled by other means, including, but not limited to, use of chromophores or fluorescent moieties, bioluminescent labels, or chemiluminescent labels.

The compounds described herein are synthesized using techniques and materials described herein and as described, for example, in Fieser and Fieser's Reagents for Organic Synthesis, Volumes 1-17 (John Wiley and Sons, 1991); Rodd's Chemistry of Carbon Compounds, Volumes 1-5 and Supplements (Elsevier Science Publishers, 1989); Organic Reactions, Volumes 1-40 (John Wiley and Sons, 1991), Larock's Comprehensive Organic Transformations (VCH Publishers Inc., 1989), March, Advanced Organic Chemistry 4th Ed., (Wiley 1992); Carey and Sundberg, Advanced Organic Chemistry 4th Ed., Vols. A and B (Plenum 2000, 2001), and Greene and Wuts, Protective Groups in Organic Synthesis 3rd Ed., (Wiley 1999) (all of which are incorporated by reference for such disclosure). General methods for the preparation of compound as described herein are modified by the use of appropriate reagents and conditions, for the introduction of the various moieties found in the formula as provided herein.

Also provided herein are compositions comprising a compound provided herein (i.e., a compound of Table 1 or a pharmaceutically acceptable salt thereof).

Also provided herein are pharmaceutical compositions, comprising a compound provided herein.

### METHODS

In another aspect, provided herein are methods of treating a cancer in a subject in need thereof, comprising administering a compound provided herein (i.e. a compound of Table 1 or a pharmaceutically acceptable salt thereof) to the subject. In some embodiments, the cancer is a HER2 positive cancer.

In some embodiments, the cancer is esophageal, stomach, colon, rectal, pancreatic, lung, breast, cervix uteri, corpus uteri, ovary, bladder, head and neck, endometrial, osteosarcoma, prostate, neuroblastoma, or a combination thereof.

In another aspect, provided herein are methods of treating viral infection, cancer, an allergic disease, or a combination thereof, in a subject in need thereof, comprising administering a compound provided herein (i.e. a compound of Table 1 or a pharmaceutically acceptable salt thereof) to the subject.

In another aspect, provided herein are methods of treating viral infection, in a subject in need thereof, comprising administering a compound provided herein (i.e. a compound of Table 1 or a pharmaceutically acceptable salt thereof) to the subject.

In another aspect, provided herein are methods of treating cancer, in a subject in need thereof, comprising administering a compound provided herein (i.e. a compound of Table 1 or a pharmaceutically acceptable salt thereof) to the subject.

In another aspect, provided herein are methods of treating an allergic disease, in a subject in need thereof, comprising administering a compound provided herein (i.e. a compound of Table 1 or a pharmaceutically acceptable salt thereof) to the subject.

In some embodiments, the viral infection comprises a hepatitis C viral (HCV) infection.

In some embodiments, provided herein is a method of modulating kinase activity in a cell, comprising contacting the cell with an amount effective to modulate kinase activity of a compound provided herein (i.e. a compound of Table 1 or a pharmaceutically acceptable salt thereof).

In some embodiments of these methods, the subject is a human.

In another aspect, provided herein are uses of a compound provided herein (i.e. a compound of Table 1 or a pharmaceutically acceptable salt thereof), a composition provided herein, or a pharmaceutical composition provided herein, in the manufacture of a medicament for the treatment of a viral infection, a cancer, or an allergic disease.

In another aspect, provided herein are pharmaceutical compositions comprising a compound provided herein and a pharmaceutically acceptable carrier.

Actual dosage levels of an active ingredient in the pharmaceutical compositions provided herein may be varied so as to obtain an amount of the active ingredient that is effective to achieve a desired therapeutic response for a particular subject, composition, or mode of administration, without being toxic to the subject.

In some embodiments, it is especially advantageous to formulate the compound in dosage unit form for ease of administration and uniformity of dosage. Dosage unit form as used herein refers to physically discrete units suited as unitary dosages for the subject to be treated; each unit containing a predetermined quantity of therapeutic compound calculated to produce the desired therapeutic effect in association with the required pharmaceutical vehicle. The dosage unit forms of the present disclosure are dictated by and directly dependent on (a) the unique characteristics of the therapeutic compound and the particular therapeutic effect to be achieved, and (b) the limitations inherent in the art of compounding/formulating such a therapeutic compound for the treatment of the diseases referred to herein in a subject in need thereof.

In one embodiment, the compounds or compositions provided herein are formulated using one or more pharmaceutically acceptable excipients or carriers. In one embodiment, the pharmaceutical compositions provided herein comprise a therapeutically effective amount of a compound provided herein and a pharmaceutically acceptable carrier.

In one embodiment, the present disclosure provides packaged pharmaceutical compositions comprising a container holding at least one therapeutically effective amount of a compound provided herein, and instructions for using the compound to treat one or more symptoms of a disease referred to herein in a subject in need thereof.

Routes of administration of any of the compositions provided herein include oral, nasal, rectal, intravaginal, parenteral, buccal, sublingual, topical, or ocular. The compounds for use as provided herein may be formulated for administration by any suitable route, such as for ocular, oral or parenteral, for example, transdermal, transmucosal (e.g., sublingual, lingual, (trans)buccal, (trans)urethral, vaginal (e.g., trans- and perivaginally), (intra)nasal and (trans)rectal), intravesical, intrapulmonary, intraduodenal, intragastrical, intrathecal, subcutaneous, intramuscular, intradermal, intra-arterial, intravenous, intrabronchial, inhalation, and topical administration.

Suitable compositions and dosage forms include, for example, drops, tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for ocular or intravesical administration and the like. It should be understood that the formulations and compositions that would be useful as provided herein are not limited to the particular formulations and compositions that are described herein.

In another aspect, provided herein are dosage forms suitable for administration to a subject in need thereof, comprising a compound provided herein (i.e. a compound of Table 1 or a pharmaceutically acceptable salt thereof).

In another aspect, provided herein are kits, comprising a composition including a compound provided herein (i.e. a compound of Table 1 or a pharmaceutically acceptable salt thereof) and instructions for use thereof. In some embodiments, the kit further includes one or more of a syringe, a vial, or a dosage form.

Those skilled in the art will recognize, or be able to ascertain using no more than routine experimentation, numerous equivalents to the specific procedures, embodiments, claims, and examples described herein. Such equivalents were considered to be within the scope of this disclosure and covered by the claims appended hereto. For example, it should be understood, that modifications in reaction conditions, including but not limited to reaction times, reaction size or volume, and experimental reagents, such as solvents, catalysts, pressures, atmospheric conditions, e.g., nitrogen atmosphere, and reducing or oxidizing agents, with art-recognized alternatives and using no more than routine experimentation, are within the scope of the present application.

It is to be understood that wherever values and ranges are provided herein, all values and ranges encompassed by these values and ranges, are meant to be encompassed within the scope of the present disclosure. Moreover, all values that fall within these ranges, as well as the upper or lower limits of a range of values, are also contemplated by the present application.

The following is a non-limiting list of embodiments of the present invention:
Embodiment 1. A compound of Formula (I): 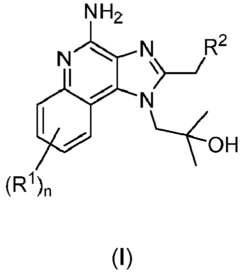 or a pharmaceutically acceptable salt thereof,
   wherein
   each occurrence of R¹ is, independently, F, Cl, Br, or I;
   R² is -O-(C₃₋₅ cycloalkyl), -O-(C₁₋₃ haloalkyl), -S-(C₁₋₃ alkyl), -S(O)(C₁₋₃ alkyl), or -S(O₂)(C₁₋₃ alkyl); and
   n is 0, 1, 2, 3, or 4.
Embodiment 2. The compound of Embodiment 1, wherein the compound of Formula (I) is a compound of Formula (II): 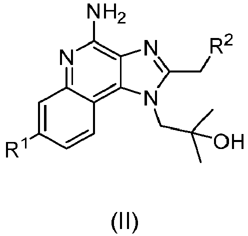 or a pharmaceutically acceptable salt thereof.
Embodiment 3. The compound of Embodiment 1, wherein R¹ is F, Cl, Br, or I.
Embodiment 4. The compound of Embodiment 1, wherein R¹ is F.
Embodiment 5. The compound of Embodiment 1, wherein R² is -O-(C₃₋₅ cycloalkyl), -O-(C₁₋₃ monohaloalkyl), -O-(C₁₋₃ dihaloalkyl), -O-(C₁₋₃ trihaloalkyl), -S-(C₁₋₃ alkyl),-S(O)(C₁₋₃ alkyl), or -S(O₂)(C₁₋₃ alkyl).
Embodiment 6. The compound of Embodiment 1, wherein R² is -O-CF₃, -O-CH₂-CF₃, —O-cyclopropyl, —S-methyl, —S-ethyl, —S(O)-methyl, —S(O)-ethyl, —S(O₂)-methyl, or -S(O₂)-ethyl.
Embodiment 7. The compound of Embodiment 1, wherein n is 0.
Embodiment 8. The compound of Embodiment 1, wherein n is 1.
Embodiment 9. The compound of Embodiment 1, wherein n is 2.
Embodiment 10. The compound of Embodiment 1, wherein the compound is: 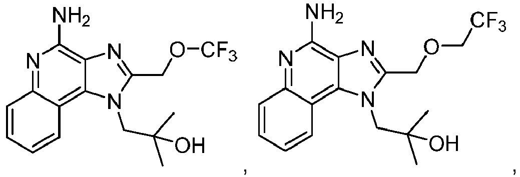 or a pharmaceutically acceptable salt thereof.
Embodiment 11. The compound of Embodiment 1, wherein the compound is: 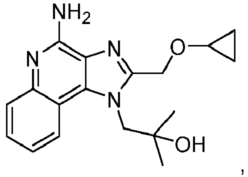 or a pharmaceutically acceptable salt thereof.
Embodiment 12. The compound of Embodiment 1, wherein the compound is: 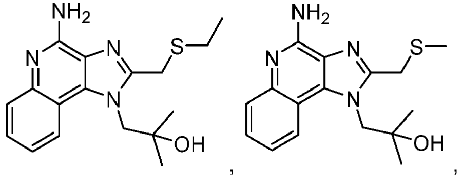 or a pharmaceutically acceptable salt thereof.
Embodiment 13. The compound of Embodiment 1, wherein the compound is: 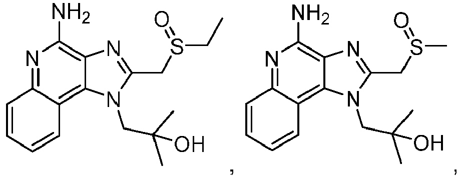 or a pharmaceutically acceptable salt thereof.
Embodiment 14. The compound of Embodiment 1, wherein the compound is: 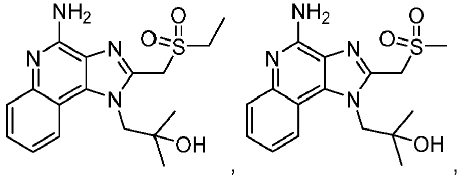 or a pharmaceutically acceptable salt thereof.
Embodiment 15. The compound of Embodiment 1, wherein the compound is: 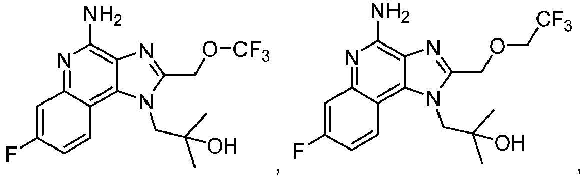 or a pharmaceutically acceptable salt thereof.
Embodiment 16. The compound of Embodiment 1, wherein the compound is: 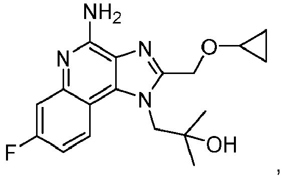 or a pharmaceutically acceptable salt thereof.
Embodiment 17. The compound of Embodiment 1, wherein the compound is: 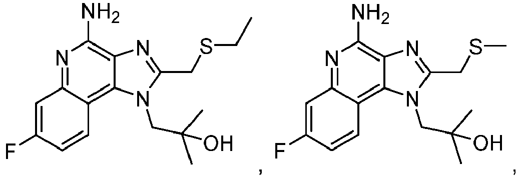 or a pharmaceutically acceptable salt thereof.
Embodiment 18. The compound of Embodiment 1, wherein the compound is: 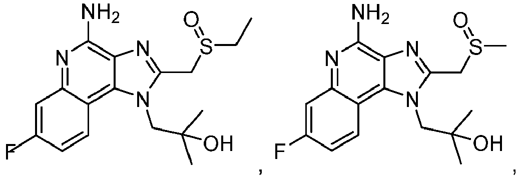 or a pharmaceutically acceptable salt thereof.
Embodiment 19. The compound of Embodiment 1, wherein the compound is: 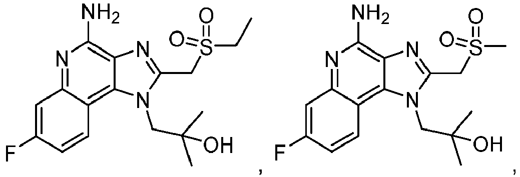 or a pharmaceutically acceptable salt thereof.
Embodiment 20. A composition comprising the compound of one of Embodiments 1-19.
Embodiment 21. A pharmaceutical composition comprising the compound of one of Embodiments 1-19, or a pharmaceutically acceptable salt thereof.
Embodiment 22. A method of treating viral infection in a subject in need thereof, comprising administering the compound of one of Embodiments 1-19 to the subject.
Embodiment 23. The method of Embodiment 22, wherein the viral infection comprises a hepatitis C viral (HCV) infection.
Embodiment 24. A method of treating cancer in a subject in need thereof, comprising administering the compound of one of Embodiments 1-19 to the subject.
Embodiment 25. A method of treating a human epidermal growth factor receptor 2 (HER2) positive cancer in a subject in need thereof, comprising administering the compound of one of Embodiments 1-19 to the subject.
Embodiment 26. The method of Embodiment 24 or Embodiment 25, wherein the cancer is esophageal, stomach, colon, rectal, pancreatic, lung, breast, cervix uteri, corpus uteri, ovary, bladder, head and neck, endometrial, osteosarcoma, prostate, or neuroblastoma.
Embodiment 27. A method of treating an allergic disease in a subject in need thereof, comprising administering the compound of one of Embodiments 1-19 to the subject.
Embodiment 28. Use of the compound of one of Embodiments 1-19, the composition of Embodiment 20, or the pharmaceutical composition of Embodiment 21, in the manufacture of a medicament for the treatment of a viral infection, a cancer, or an allergic disease.
Embodiment 29. A dosage form suitable for administration to a subject in need thereof, comprising the compound of one of Embodiments 1-19.
Embodiment 30. A kit, comprising the compound of one of Embodiments 1-19 and instructions for use thereof.

The following examples further illustrate aspects of the present disclosure. However, they are in no way a limitation of the teachings or present disclosure as set forth herein.

### EXAMPLES

In the synthetic schemes shown below, unless otherwise indicated all temperatures are set forth in degrees Celsius and all parts and percentages are by weight. Reagents and solvents are purchased from commercial suppliers and are used without further purification unless otherwise indicated. Anhydrous tetrahydrofuran (THF) and N,N-dimethylforamide (DMF) are purchased from commercial sources and used as received.

The reactions set forth below are done generally under a positive pressure of argon or nitrogen at an ambient temperature (unless otherwise stated) in anhydrous solvents. Glassware is oven dried or heat dried. The reactions are assayed by TLC or analyzed by LC-MS and terminated as judged by the consumption of starting material. Analytical thin layer chromatography (TLC) is performed on glass plates pre-coated with silica gel 60 F254 0.25 mm plates (EM Science), and visualized with UV light (254 nm) or heating with commercial ethanolic phosphomolybdic acid. Preparative thin layer chromatography (TLC) is performed on glass-plates pre-coated with silica gel 60 F254 0.5 mm plates (20 × 20 cm, from commercial sources) and visualized with UV light (254 nm).

Work-up of the reactions is typically done by doubling the reaction volume with the reaction solvent or extraction solvent and then washing with the indicated aqueous solutions using 25 % by volume of the extraction volume unless otherwise indicated. Product solutions are dried over anhydrous Na₂SO₄ or Mg₂SO₄ prior to filtration and evaporation of the solvents under reduced pressure on a rotary evaporator and noted as solvents removed in *vacuo.* Column chromatography is completed under positive pressure using 230-400 mesh silica gel.

¹H-NMR spectra and ¹³C-NMR are recorded on a Varian Mercury-VX400 instrument operating at 400 MHZ. NMR spectra are obtained as CDCl₃ solutions (reported in ppm), using chloroform as the reference standard (7.27 ppm for ¹H and 77.00 ppm for ¹³C), CD₃OD (3.4 and 4.8 ppm for ¹H and 49.3 ppm for ¹³C), DMSO-d₆ (2.49 ppm for ¹H), or internal tetramethylsilane (0.00 ppm for ¹H) when appropriate. Other NMR solvents are used as needed.

### Example 1: Synthesis of common intermediate A.

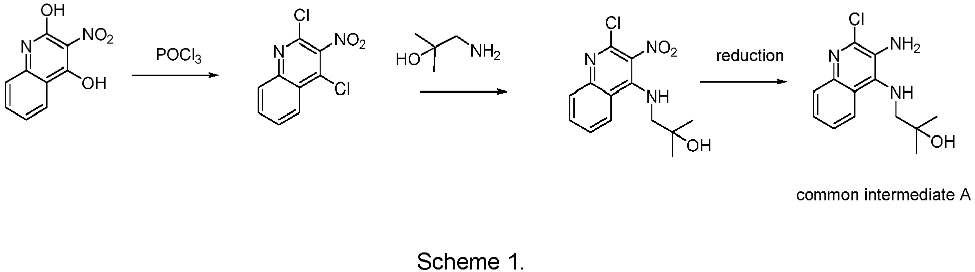

### Example 2: Synthesis of common intermediate B.

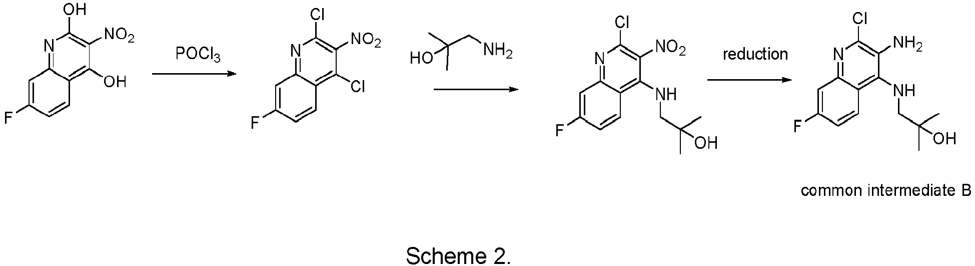

### Example 3: Synthesis of Compound 1.

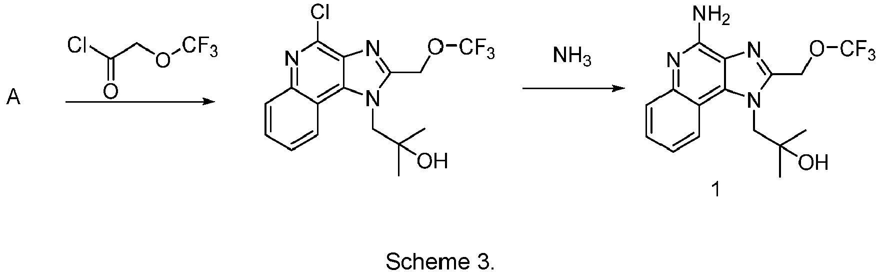

### Example 4: Synthesis of Compound 2.

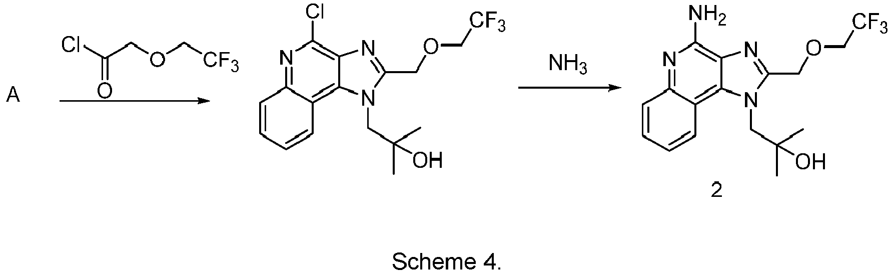

### Example 5: Synthesis of Compound 3.

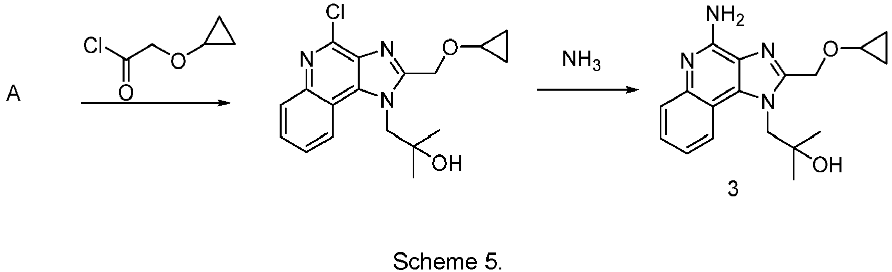

### Example 6: Synthesis of Compound 4.

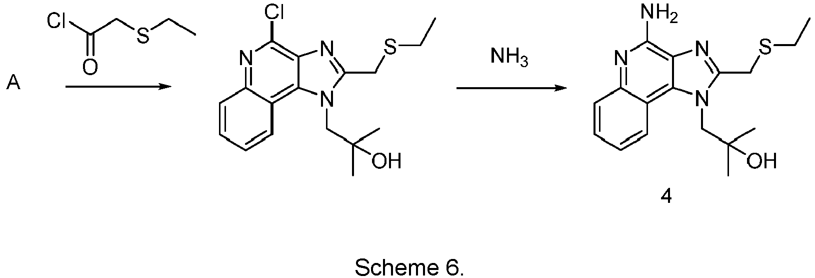

### Example 7: Synthesis of Compound 5.

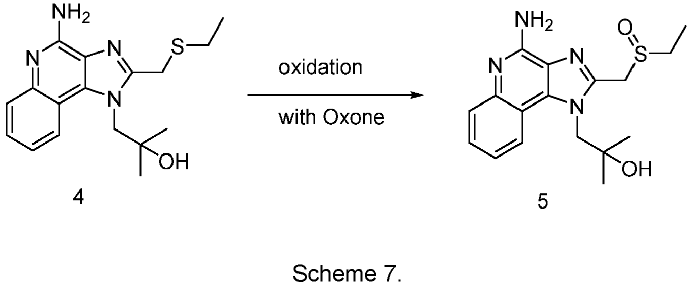

### Example 8: Synthesis of Compound 6.

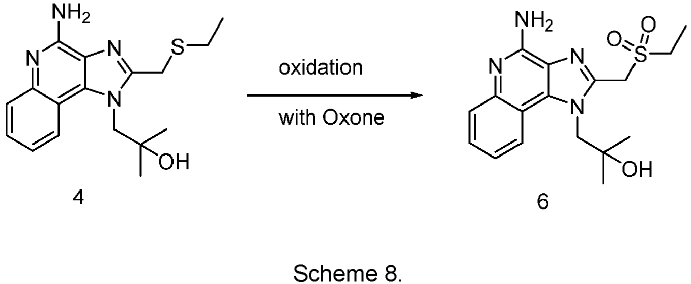

### Example 9: Synthesis of Compound 10.

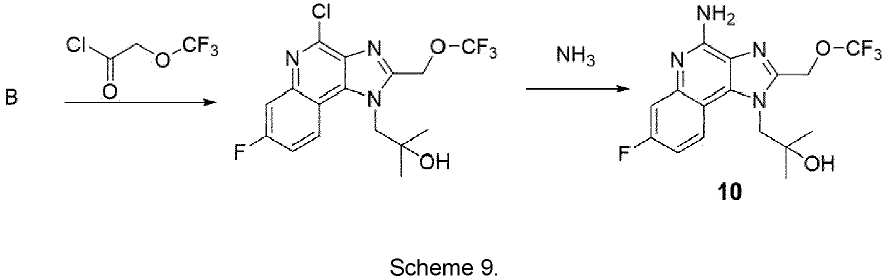

### Example 10: Synthesis of Compound 11.

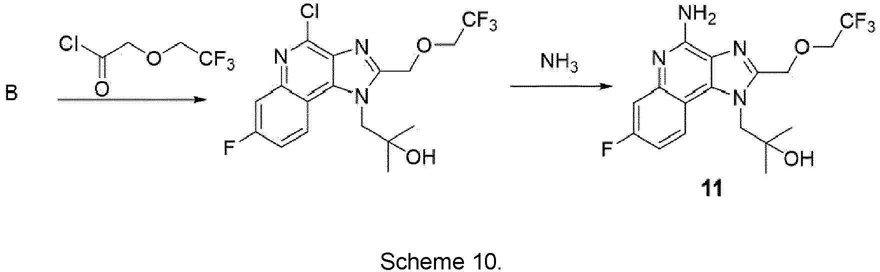

### Example 11: Synthesis of Compound 12.

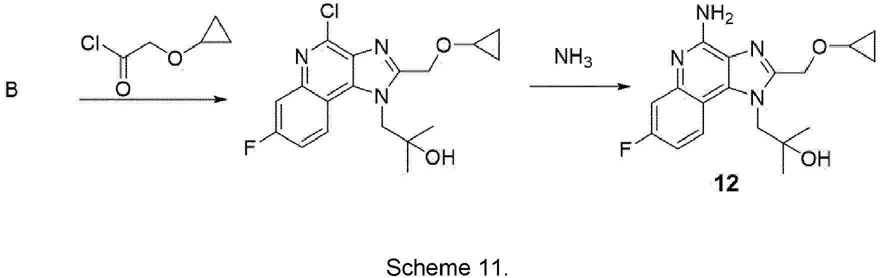

### Example 12: Synthesis of Compound 13.

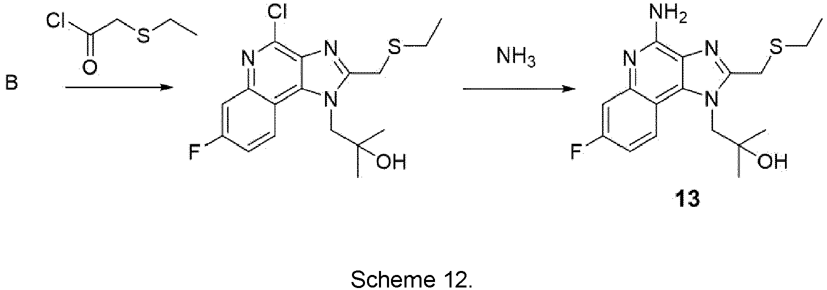

### Example 13: Synthesis of Compound 14.

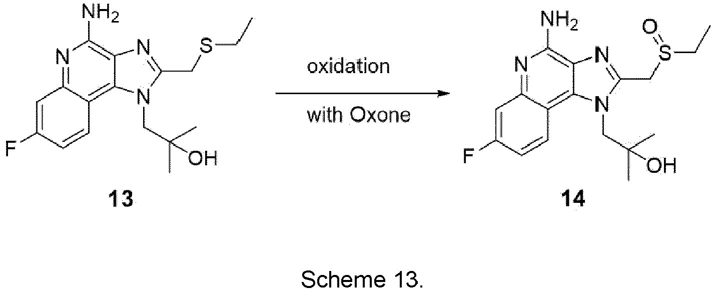

### Example 14: Synthesis of Compound 15.

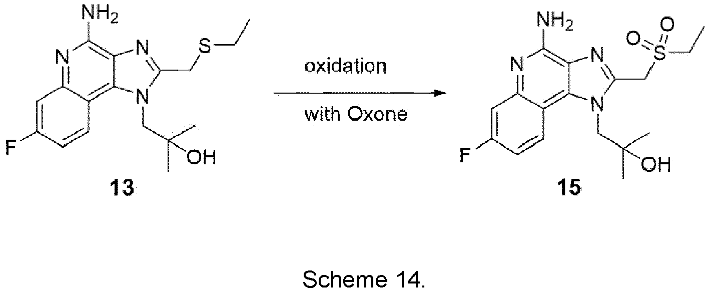

### Example 15: TLR7/8 Agonist PK Study

SPF SD rats were purchased from BioLASCO Taiwan Co. Ltd. Animals were randomly assigned to five groups with 3 males and 3 females in each group before dosing. The body weight variation of animals used were in an interval within ± 20 percent of the mean weight for each gender. The basic design was as shown in Table 2.

**Table 2. TLR7/8 agonist PK Study Design**

| **Group** | **Compound** | **Dose (mg/kg)** | **Dose Conc. (mg/mL)** | **Dose Volume (mL/kg)** | **Dosing Route** | **No. of Animals** |
|---|---|---|---|---|---|---|
| 1 | Resiquimod | 0.08 | 0.04 | 2.0 | *i.v.* | 6 (3 male & 3 female) |
| 2 | Compd. 3 | 0.08 | 0.04 | 2.0 | *i.v.* | 6 (3 male & 3 female) |
| 3 | Compd. 6 | 0.08 | 0.04 | 2.0 | *i.v.* | 6 (3 male & 3 female) |
| 4 | Compd. 4 | 0.08 | 0.04 | 2.0 | *i.v.* | 6 (3 male & 3 female) |

Blood samples (200-350 µL/time point) were obtained through tail vein at pre-dose and 2 min, 5 min, 15 min, 30 min, 1 hr, 2 hr, 4 hr, 6 hr, 8 hr, 12 hr and 24 hr post dosing. The blood samples were collected into tubes containing K₂EDTA and maintained on ice no more than 30 minutes, then centrifuged (1500-1750 ×g, 15 min, 2-8 °C) for plasma harvest.

The study samples were prepared and analyzed in parallel using liquid chromatography-tandem mass spectrometry. In pharmacokinetics evaluation following the determined concentrations in rat plasma, pharmacokinetic parameters were calculated using WinNonlin (Pharsight Corporation, version 6.3) with non-compartmental methods.

Results are shown in Fig. 1, and below in Table 3 (italicized values showed significant difference (P<0.05) when compared with the resiquimod group). Resiquimod is a Toll-like receptor (TLR) agonist that activates the immune system.

**Table 3. Pharmacokinetic Parameters of TLR7/8 Agonists**

| **Dose Route** | **Dose Level (mg/kg)** | **Compound** | **C₀ (ng/mL)** | **T_{1/2} (hr)** | **Cl (mL/hr/kg)** | **Vdₛₛ (mL/kg)** |
|---|---|---|---|---|---|---|
| *i.v.* | 0.08 | Resiquimod | 257.54 ±47.57 | 8.46 ±1.72 | 1,297.98 ±90.13 | 4,672.66 ±1,101.60 |
| *i.v.* | 0.08 | Compd. 3 | 236.85 ±44.25 | 7.53 ±1.03 | *713.06* ±59.79 | 3,129.26 ±427.78 |
| *i.v.* | 0.08 | Compd. 6 | 224.00 ±53.89 | 6.46 ±1.01 | 1,025.00 ±119.21 | 1,822.00 ±348.64 |
| *i.v.* | 0.08 | Compd. 4 | 257.54 ±47.57 | 8.46 ±1.72 | 1,297.98 ±90.13 | 4,672.66 ±1,101.60 |

| **Dose Route** | **Dose Level (mg/kg)** | **Compound** | **MRT₀₋ₜ (hr)** | **MRT_{inf} (hr)** | **AUC₀₋ₜ (hr*ng/mL)** | **AUC_{0-inf} (hr*ng/mL)** |
|---|---|---|---|---|---|---|
| *i.v.* | 0.08 | Resiquimod | 1.87 ±0.16 | 3.87 ±1.20 | 62.50 ±4.49 | 66.23 ±4.81 |
| *i.v.* | 0.08 | Compd. 3 | *3.15* ±*0.18* | *4.90* ±*0.87* | *117.61* ±*8.92* | *124.35* ±*9.96* |
| *i.v.* | 0.08 | Compd. 6 | 1.28 ±0.19 | 1.71 ±0.22 | 81.60 ±8.49 | 82.80 ±8.29 |
| *i.v.* | 0.08 | Compd. 4 | 1.87 ±0.16 | 3.87 ±1.20 | 62.50 ±4.49 | 66.23 ±4.81 |

### Example 16: TLR7/8 Agonist Potency Assay in HEK-Blue hTLR7 Cell

HEK-Blue hTLR7 cells were seeded at 3×10⁴ cell/well and treated with nine concentrations of positive control (CL264) and three compounds (resiquimod, compound 2, compound 3). HEK-Blue TLR7 cells can serve to measure the bioactivity of TLR7 through the secretion of embryonic alkaline phosphatase (SEAP) upon NF-κB activation following TLR7 stimulation. Water was used as negative control. After about 16 hrs incubation at 37 °C in 5% CO₂, the SEAP was determined by using a spectrophotometer at 635 nm.

The dose-response curves were fit with four parameter logistic curve (SigmaPlot, version 9.0). The calculated EC₅₀-values for positive control (CL264) and the tested compounds are shown below in Table 4 (italicized values showed significant difference (P<0.05) when compared with the resiquimod group).

**Table 4. TLR7 Agonistic EC₅₀ of TLR7/8 Agonists**

| Compound | EC₅₀ (ng/mL) |
|---|---|
| CL264 | 260.2 ±12.4 |
| Resiquimod | 59.9 ±9.0 |
| Compound 2 | *36.2* ±*3.6* |
| Compound 3 | *40.5* ±*2.2* |

### Example 17: TLR7/8 Agonist Potency Assay in HEK-Blue hTLR8 Cell

HEK-Blue hTLR8 cells were seeded at 3×10⁴ cell/well and treated with nine concentrations of positive control (ssRNA40) and four test articles (resiquimod, compound 2, compound 3). HEK-Blue hTLR8 cells can serve to measure the bioactivity of TLR8 through the secretion of embryonic alkaline phosphatase (SEAP) upon NF-κB activation following TLR8 stimulation. Water was used as negative control. After about 16 hrs incubation at 37 °C in 5% CO₂, the SEAP was determined by using a spectrophotometer at 635 nm.

The dose-response curves were fit with four parameter logistic curve (SigmaPlot, version 9.0). The calculated EC₅₀-values for positive control (ssRNA40) and the tested compounds were shown below in Table 5 (italicized values showed significant difference (P<0.05) when compared with the resiquimod group).

**Table 5. TLR8 Agonistic EC₅₀ of TLR7/8 Agonists**

| Compound | EC₅₀ (ng/mL) |
|---|---|
| ssRNA40 | 2,722.5 ±61.1 |
| resiquimod | 375.9 ±1.9 |
| Compound 2 | *591.2* ±*20.2* |
| Compound 3 | *515.5* ±*10.5* |

## Claims

1. A composition comprising a compound for use in influencing, reducing, inhibiting or modulating kinase activity in a cell of a subject, wherein the compound is a compound of Formula (I): 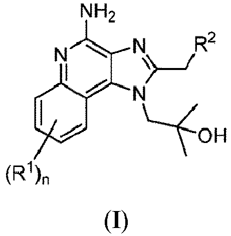 or a pharmaceutically acceptable salt thereof,
wherein
each occurrence of R¹ is, independently, F, Cl, Br or I;
R² is -O-CF₃, -O-CH₂-CF₃, —O-cyclopropyl, —S-methyl, —S-ethyl, —S(O)-methyl, —S(O)-ethyl, -S(O₂)-methyl, or -S(O₂)-ethyl; and
n is 0 or 1.

2. The composition for use according to claim 1, wherein R¹ is F.

3. The composition for use according to claim 1, wherein the composition comprises a compound selected from: 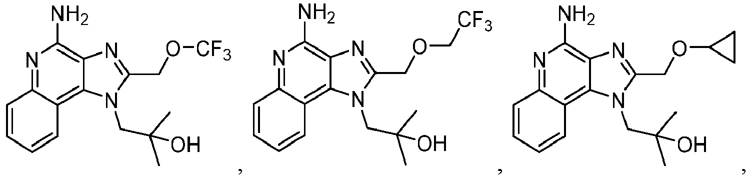 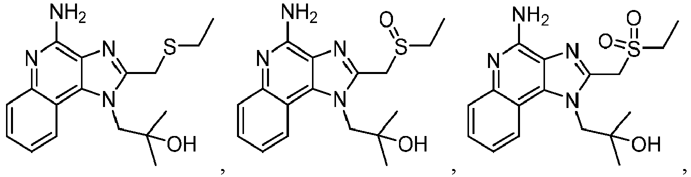 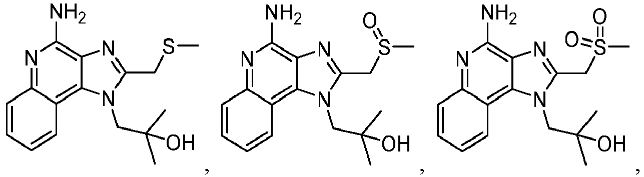 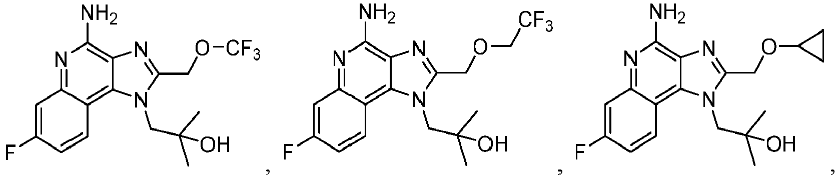 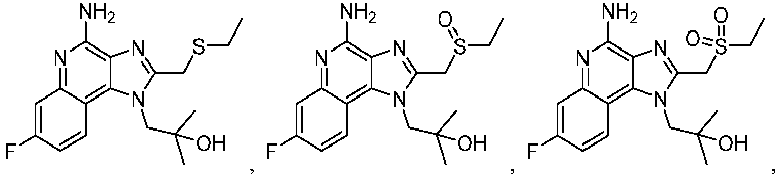 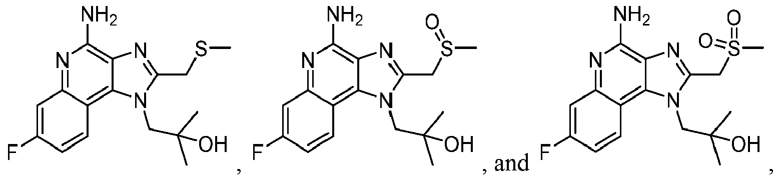 or a pharmaceutically acceptable salt thereof.

4. The composition for use according to claim 1, wherein the subject has a disease or disorder associated with kinase activity.

5. The composition for use according to claim 2, wherein the subject has a disease or disorder associated with kinase activity.

6. The composition for use according to claim 3, wherein the subject has a disease or disorder associated with kinase activity.

7. The composition for use according to claim 6, wherein the compound is 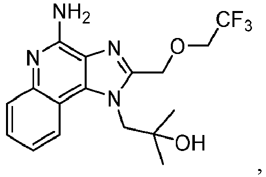 or a pharmaceutically acceptable salt thereof.

8. The composition for use according to claim 6, wherein the compound is 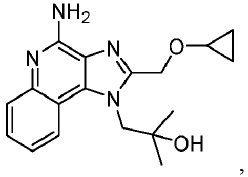 or a pharmaceutically acceptable salt thereof.

9. The composition for use according to any one of claims 4-8, wherein the subject has a disease or disorder associated with TLR7 kinase activity and/or TLR8 kinase activity.

10. The composition for use according to claim 9, wherein the disease or disorder is a viral infection, cancer or an allergic disease.

11. The composition for use according to claim 9, wherein the disease or disorder is a hepatitis C viral infection.

12. The composition for use according to claim 9, wherein the disease or disorder is a human epidermal growth factor receptor 2 (HER2) positive cancer.

13. The composition for use according to claim 9, wherein the disease or disorder is a cancer selected from esophageal, stomach, colon, rectal, pancreatic, lung, breast, cervix uteri, corpus uteri, ovary, bladder, head and neck, endometrial, osteosarcoma, prostate, and neuroblastoma.

14. The composition for use according to any one of claims 1-13, wherein the subject is a human.
